(19) Europäisches Patentamt
European Patent Office
Office européen des brevets

(11) Veröffentlichungsnummer: **0 229 128**
**B1**

(12) **EUROPÄISCHE PATENTSCHRIFT**

(45) Veröffentlichungstag der Patentschrift: **30.05.90**

(51) Int. Cl.⁵: **C 07 F 9/10**, A 61 K 31/685

(21) Anmeldenummer: **86904150.9**

(22) Anmeldetag: **02.07.86**

(86) Internationale Anmeldenummer:
**PCT/EP86/00390**

(87) Internationale Veröffentlichungsnummer:
**WO 87/00173 15.01.87 Gazette 87/01**

# (54) GLYCERO-3(2)-PHOSPHO-L-SERINDERIVATE UND DIESE ENTHALTENDE PHARMAZEUTISCHEN PRÄPARATE.

(30) Priorität: **03.07.85 DD 278230**
**03.07.85 DD 278231**
**15.07.85 DD 278560**
**15.07.85 DD 278561**
**15.07.85 DD 278562**

(43) Veröffentlichungstag der Anmeldung:
**22.07.87 Patentblatt 87/30**

(45) Bekanntmachung des Hinweises auf die Patenterteilung:
**30.05.90 Patentblatt 90/22**

(84) Benannte Vertragsstaaten:
**AT BE CH DE FR GB IT LI LU NL SE**

(56) Entgegenhaltungen:
**DD-A- 222 595**
**GB-A-2 057 872**
**D.R.Hoffman et al. Research Communications in Chemical Pathology and Pharmacology 44, 293-306 (1984)**
**Honma, Y., Kasukabe, T., Hozumi, M., Tsushima, S., and Nomura, H. (1981) Induction of differentiation of cultured human and mouse myeloid leukemia cells by alkyl-lysophospholipids. Cancer Res. 41, Seiten 3211-3216**

(73) Patentinhaber: **CL PHARMA AKTIENGESELLSCHAFT**
**St. Peter-Strasse 25**
**A-4021 Linz (AT)**

(72) Erfinder: **BRACHWITZ, Hans Akademie der Wissenschaften**
**Otto-Nuschke-Strasse 22/23**
**D-x 1086 Berlin (DD)**
Erfinder: **LANGEN, Peter Akademie der Wissenschaften**
**Otto-Nuschke-Strasse 22/23**
**D-x 1086 Berlin (DD)**
Erfinder: **LEHMANN, Christine Akademie der Wissenschaften**
**Otto-Nuschke-Strasse 22/23**
**D-x 1086 Berlin (DD)**
Erfinder: **MATTHES, Eckart Akademie der Wissenschaften**
**Otto-Nuschke-Strasse 22/23**
**D-x 1086 Berlin (DD)**
Erfinder: **SCHILDT, Jürgen Akademie der Wissenschaften**
**Otto-Nuschke-Strasse 22/23**
**D-x 1086 Berlin (DD)**

**EP 0 229 128 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(72) Erfinder: **FICHTNER, Iduna Akademie der Wissenschaften**
**Otto-Nuschke-Strasse 22/23**
**D-x 1086 Berlin (DD)**
Erfinder: **HERMETTER, Albin Technische Universität Graz**
**Schlögelgasse 9**
**A-8010 Graz (AT)**
Erfinder: **PALTAUF, Friedrich Technische Universität Graz**
**Schlögelgasse 9**
**A-8010 Graz (AT)**

(74) Vertreter: **Kunz, Ekkehard, Dr.**
**Chemie Holding AG Patentwesen St. Peter-Strasse 25**
**A-4021 Linz (AT)**

## Beschreibung

Die Erfindung betrifft neue Glycero-3(2)-phospho-L-serinderivate der allgemeinen Formel

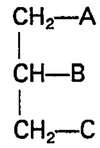

I.

in der

A unsubstituiertes oder ein- oder mehrfach durch Halogen, Hydroxy, Alkoxy oder Cyano substituiertes ($C_5$—$C_{30}$)-Alkoxy oder unsubstituiertes oder ein- oder mehrfach durch Halogen, Hydroxy, Alkoxy oder Cyano substituiertes ($C_5$—$C_{30}$)-Alkenoxy, wobei eine Doppelbindung des Alkenoxyrestes nicht von dem an den Sauerstoff gebundenen C-Atom ausgeht, Halogen oder eine Gruppe der allgemeinen Formel

$$—O—(CH_2)_n—CF_3 \qquad II$$

worin

n für 0 oder eine ganze Zahl 1, 2 oder 3 steht, bedeutet, einer der beiden Reste

B und C, der gleich wie A oder davon verschieden ist, eine der für A angegebenen Bedeutungen hat oder Wasserstoff bedeutet und der jeweils andere Rest die Phosphatidyl-L-serin-Gruppe der Formel

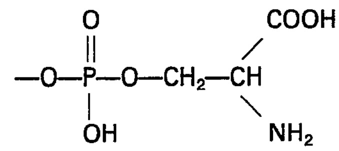

III

darstellt, mit der Maßangabe, daß mindestens ein Rest A, B oder C ($C_5$—$C_{30}$)-Alkoxy oder ($C_5$—$C_{30}$)-Alkenoxy bedeutet; und die pharmazeutisch annehmbaren Salze von Verbindungen der allgemeinen Formel I mit Basen, Verfahren zu ihrer Herstellung, diese Verbindungen enthaltende pharmazeutische Präparate sowie ihre Verwendung in Arzneimitteln mit cytostatischer Wirkung.

Die Glycerophospho-L-serinderivate der allgemeinen Formel I verfügen in jedem Fall im Serin-Teil des Moleküls über ein Chiralitätszentrum und können in Abhängigkeit von der Bedeutung der Rest A, B und C ein zweites Chiralitätszentrum im Glycerin-Teil des Moleküls aufweisen. Gegenstand der Erfindung und von der allgemeinen Formel I umfaßt sind demnach auch alle möglichen chiralen und diastereomeren Formen der erfindungsgemäßen Verbindungen.

Der in dieser Beschreibung verwendete Ausdruck "Alkoxy" bezieht sich auf geradkettige, einfach oder mehrfach verzweigte Alkylethergruppen, die bevorzugt 14—20 Kohlenstoffatome und ganz bevorzugt 16—18 Kohlenstoffatome aufweisen. Beispiele für bevorzugte Alkoxyreste sind Tetra-, Penta-, Hexa-, Hepta-, Octa-, Nonadecyloxy, Eicosyloxy bzw. deren verzweigte Analogen. Der Ausdruck "Alkenyloxy" steht für geradkettige, einfach oder mehrfach verzweigte, einfach oder mehrfach ungesättigte Alkenylethergruppen, die bevorzugt 14—20 und ganz bevorzugt 16—18 Kohlenstoffatome aufweisen, wobei in diesen Alkenylethergruppen Enolethergruppierungen, bei denen eine olefinische Doppelbindung von dem an den Sauerstoff gebundenen Kohlenstoffatom ausgeht, ausdrücklich ausgenommen sind. Sowohl die oben näher beschriebenen Alkoxy-als auch Alkenoxyreste A, B und C können ein- oder mehrmals, vorzugsweise einmal, substituiert sein, wobei als solche Substituenten bevorzugt Halogen, Hydroxy, Alkoxy oder Cyano in Frage kommen.

Die Bezeichnung "Halogen" bezieht sich auf die vier Halogene Chlor, Brom, Jod und Fluor, wobei Chlor und Fluor besonders bevorzugt sind.

In einer bevorzugten Klasse von Verbindungen der allgemeinen Formel I bedeutet C die Phosphatidyl-L-serin-Gruppe der Formel III, einer der Reste A und B Alkoxy oder Alkenoxy und der jeweils andere Rest Halogen.

Eine weitere bevorzugte Klasse betrifft Verbindungen der allgemeinen Formel I, worin C die Phosphatidyl-L-serin-Gruppe der Formel III, einer der Reste A und B Alkoxy oder Alkenoxy und der jeweils andere einen endständig fluorierten Alkoxyrest der Formel II bedeutet.

Bevorzugt sind auch solche Verbindungen der allgemeinen Formel I, worin A Alkoxy oder Alkenoxy, B Wasserstoff oder Alkoxy und C die Phosphatidyl-L-serin-Gruppe der Formel III bedeutet.

Schließlich ist auch jene Klasse von Verbindungen der allgemeinen Formel I bevorzugt, worin B die Phosphatidyl-L-serin-Gruppe der Formle III, einer der Reste A und C Alkoxy oder Alkenoxy und der jeweils andere halogen bedeutet.

Besonders bevorzugte Einzelverbindungen der Erfindung sind:

1-O-Hexadecyl-2-chlor-2-desoxy-glycero-3-phospho-L-serin

1-O-Hexadecyl-2-fluor-2-desoxy-glycero-3-phospho-L-serin

1-Chlor-1-desoxy-3-O-hexadecyl-glycero-2-phospho-L-serin
1-Chlor-1-desoxy-2-O-hexadecyl-glycero-3-phospho-L-serin
1-O-Hexadecyl-2-desoxy-glycero-3-phospho-L-serin
1,2-Di-O-hexadecyl-glycero-3-phospho-L-serin
1-O-(2,2,2-Trifluorethyl)-2-O-hexadecyl-glycero-3-phospho-L-serin
1-O-Hexadecyl-2-O-(2,2,2-trifluorethyl)-glycero-3-phospho-L-serin
1-Chlor-1-desoxy-2-O-octadecyl-glycero-3-phospho-L-serin
1-O-Octadecyl-2-(2,2,2-trifluorethyl)-glycero-3-phospho-L-serin
1-O-Octadecyl-2-chlor-2-desoxy-glycero-3-phospho-L-serin.

Die Glycero-3(2)-phospho-L-serinderivate der allgemeinen Formel I und deren pharmazeutisch verträgliche Salze werden erfindungsgemäß nach an sich bekannten Methoden, vorzugsweise dadurch hergestellt, daß man

a) ein Glycero-3(2)-phosphorsäurederivat der allgemeinen Formel

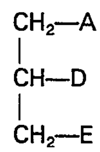

IV

in der

A wie in Formel definiert ist, einer der beiden Reste

D und E eine der in Formel I für A angegebenen Bedeutungen hat oder Wasserstoff ist und der jeweils andere Rest eine Gruppe der allgemeinen Formel

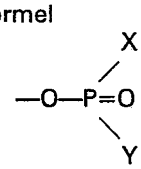

V

worin

X und Y entweder gleich sind und Hydroxy oder Halogen bedeuten oder Y für niederes Alkoxy oder Aryloxy steht, darstellt, mit der Maßgabe, daß mindestens ein Rest A, D oder E ($C_5$—$C_{30}$)-Alkoxy oder ($C_5$—$C_{30}$)-Alkenoxy bedeutet, oder dessen Salze mit einem geschützten L-Serinderivat der allgemeinen Formel

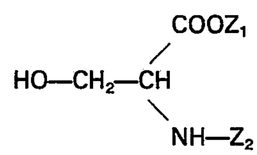

VI

in der

$Z_1$ eine Carboxyl- und

$Z_2$ eine Aminoschutzgruppe bedeutet, vorzugsweise in Anwesenheit eines Kondensationsmittels, umsetzt, anschließend in beliebiger Reihenfolge oder gleichzeitig die Schutzgruppen $Z_1$ und $Z_2$ abspaltet und gegebenenfalls entstandene Glycerophosphorsäureester oder- halogenide verseift, oder

b) einen Glycero-3(2)-phosphorsäureester der allgemeinen Formel

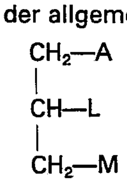

VII

in der

A wie in Formel I definiert ist, einer der beiden Reste

L und M eine der in Formel I für A angegebenen Bedeutungen hat oder Wasserstoff ist und der jeweils andere Rest eine Gruppe der allgemeinen Formel

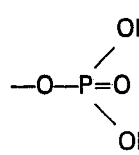

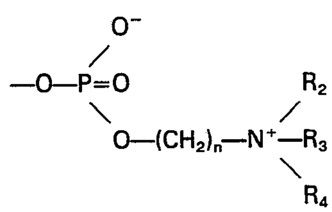

VIIIa oder VIIIb

worin

$R_1$ gegebenenfalls durch Hydroxy oder Halogen substituiertes ($C_1$—$C_6$)-Alkyl,

$R_2$, $R_3$ und $R_4$, die gleich oder verschieden sind, unabhängig voneinander Wasserstoff oder Methyl und

n eine ganze Zahl von 1—6 bedeuten, darstellt, mit der Maßgabe, daß mindestens ein Rest A, L oder M ($C_5$—$C_{30}$)-Alkoxy oder ($C_5$—$C_{30}$)-Alkenoxy bedeutet, mit L-Serin in Gegenwart von Phospholipase D zur Umsetzung bringt und die entstandenen Verbindungen der allgemeinen Formel I oder deren Salze isoliert, und

c) erwünschtenfalls eine nach Verfahrensvariante a) oder b) erhaltene Verbindung der allgemeinen Formel I oder ein pharmazeutisch nicht annehmbares Salz davon in ein pharmazeutisch annehmbares Salz überführt.

Gemäß Verfahrensvariante a) können die Verbindungen der allgemeinen Formel I demnach aus Glycero-3(2)-phosphorsäurederivaten der Formel IV und entsprechend geschützten L-Serinen der Formel VI nach an sich bekannten, dem Fachmann geläufigen Methoden hergestellt werden. Beispielsweise ist die Herstellung von Phosphatidylserinen mit gesättigten und ungesättigten Acylresten durch Kondensation von Phosphatidsäuren mit an der Amino- und Carboxylgruppe geschützten Serinen oder durch Umsetzung von Diacylglyceroljodhydrinen mit geschützten O-Phosphoserinen, wobei die Schutzgruppen der als Zwischenprodukte entstandenen geschützten Phosphatidylserine entweder gleichzeitig oder nacheinander mit geeigneten Methoden abgespalten werden, bekannt. Anstelle der Phosphatidsäuren wurden auch Phosphatidsäurechloride zur Reaktion eingesetzt. In analoger Weise lassen sich auch alkyl- und alkylensubstituierte Glycerophosphoserine synthetisieren (A. J. Slotboom und P. P. Bonsen, Chem. Phys. Lipids 5(1970), 301—398; M. Kates, in: E. D. Korn (Herausgeber) Methodes in Membrane Biology, Vol. 8, Plenum Press, New York, 1977, S. 119 ff.; H. Eibl, Chem. Phys. Lipids 26 (1980) 405—429; A. Hermetter, F. Paltauf und H. Hauser, Chem. Phys. Lipids 30 (1982) 35—45).

Zur Herstellung der neuen Glycero-3(2)-phospho-L-serinderivate der allgemeinen Formel I werden bevorzugt entsprechend substituierte Glycero-3(2)-phosphorsäuren der Formel IV eingesetzt, bei denen ein Rest D oder E eine Gruppe der Formel V, worin X und Y Hydroxy bedeuten, darstellt.

Anstelle der freien Säuren lassen sich aber auch die entsprechenden Phosphorsäuredihalogenid (X und Y in V: Halogen), vorzugsweise die Phosphorsäuredichloride, verwenden, die man durch Umsetzung der entsprechend substituierten Glycerole mit Phosphoroxyhalogeniden erhält. Bei der Umsetzung dieser Phosphorsäuredihalogenide der Formel IV (X und Y in V: Halogen) mit einem geschützten Serin vom Typ VI entstehen als Zwischenprodukte an der Serin-Gruppe geschützte Glycero-phospho-L-serin-chloride von Verbindungen der allgemeinen Formel I, die vor oder nach ihrer Isolierung und vor, während oder nach Entfernung der Schutzgruppen in die Verbindungen der allgemeinen Formel I oder deren Salze umgewandelt werden können.

Eine weitere Variante des erfindungsgemäßen Verfahrens besteht darin, daß man die geschützten Serine der Formel VI mit Glycerophosphorsäureester-Halogeniden der Formel IV zur Reaktion bringt, bei denen ein Rest D oder E eine solche Gruppe der Formel V ist, worin X Halogen, vorzugsweise Chlor, und Y niederes Alkoxy oder Aryloxy bedeutet. Die als Zwischenprodukte erhaltenen, an der Serin-Gruppe geschützten Alkyl- bzw. Arylester von Verbindungen der allgemeinen Formel I können nach üblichen chemischen Methoden durch Hydrolyse und Abspaltung der Schutzgruppen in die Säuren der allgemeinen Formel I oder deren Salze umgewandelt werden.

Glycerophosphorsäurealkyl(-aryl)ester-Halogenide der Formel IV sind durch Umsetzung entsprechend substituierter Glycerole mit Phosphorsäurealkyl (bzw.-aryl)ester-Dihalogeniden leicht zugänglich.

Als geschützte L-Serinderivate eignen sich zur erfindungsgemäßen Umsetzung solche Verbindungen der Formel VI, worin $Z_1$ eine in der Peptidchemie gebräuchliche, beispielsweise durch katalytische Hydrogenolyse, Hydrazinolyse, Behandlung mit HCl, Natriumthiophenolat oder durch Hydrolyse leicht abspaltbare Carboxyl-Schutzgruppe, wie Benzyl, tert. Butyl, Phtalimidomethyloxy, Isopropyl, Benzhydryl oder dergleichen, und $Z_2$ eine in der Peptidchemie übliche, beispielsweise durch katalytische Hydrogenolyse, Hydrazinolyse, Behandlung mit HCl oder Ameisensäure entfernbare Aminoschutzgruppe bedeutet. Solche Aminoschutzgruppen sind beispielsweise Acylgruppen, wie Formyl, Acetyl, Trifluoracetyl; Alkoxycarbonylgruppen, wie Ethoxycarbonyl, tert. Butoxycarbonyl, β,β,β-Trichlorethoxycarbonyl, β-Jodethoxycarbonyl; Aralkoxycarbonyl wie Benzyloxycarbonyl, p-Methoxybenzyloxycarbonyl; Aryloxycarbonyl wie Phenoxycarbonyl; Silylgruppen wie Trimethylsilyl; und andere Gruppen wie Trityl, Tetrahydropyranyl, Vinyloxycarbonyl, O-Nitrophenylsulfenyl, Diphenylphosphynil, p-Toluolsulfonyl, Benzyl und dergleichen.

An sich können die Schutzgruppen $Z_1$ und $Z_2$ beliebig gewählt werden, doch ist es in der Regel vorteilhaft, zum Schutz des L-Serin solche Schutzgruppenkombinationen zu wählen, die in einem Reaktionsschritt abgespalten werden können, indem man beispielsweise als geschütztes Serinderivat der Formel VI N-tert.-Butyloxy-L-serin-benzhydrylester und dergleichen verwendet.

Die Umsetzung von Verbindungen der Formel IV mit jenem der Formel VI zu den gemäß Verfahrensschritt a) als Zwischenprodukte gebildeten, an der Serin-Gruppe noch geschützten Derivaten der Verbindungen der allgemeinen Formel I wird im allgemeinen so durchgeführt, daß man ein Glycero-3(2)-phosphorsäurederivat der Formel IV, vorzugsweise ein gut getrocknetes Salz einer Glycero-3(2)-phosphorsäure der Formel IV, beispielsweise das Pyridiniumsalz, mit dem geschützten Serin der Formel VI im Molverhältnis von 1:1 bis 1:5 in Gegenwart einer starken Base, wie Pyridin, Triethylamin, Hüning-Base und dergleichen, und gegebenenfalls eines zusätzlichen inerten, apolaren organischen Lösungsmittels, wie Chloroform, Essigester, Diethylether, Diisopropylether, Benzol, Chlorbenzol, Tetrahydrofuran und dergleichen, umsetzt.

Die Umsetzung wird vorzugsweise in Anwesenheit eines geeigneten Kondensationsmittels, z.B. 2,4,6-Triisopropylbenzolsulfochlorid, bei Raumtemperatur oder Temperaturen, die knapp darüber oder darunter liegen, durchgeführt.

Die Abspaltung der Schutzgruppen aus den bei dieser Umsetzung erhaltenen geschützten Zwischenprodukten kann je nach den gewählten Schutzgruppen mit den jedem Fachmann geläufigen Methoden erfolgen. Beispielsweise können Zwischenprodukte in Form von durch Alkoxycarbonyl geschützten Benzhydrylestern durch Behandlung mit HCl, vorzugsweise durch Einleiten von HCl in Lösungen der Zwischenprodukte in organischen Lösungsmitteln, unter Abspaltung beider Schutzgruppen in Verbindungen der Formel I übergeführt werden, eventuell vorhandene Trityl-Schutzgruppen können durch Hydrogenolyse, andere durch Hydrolyse, Hydrazinolyse und dergleichen entfernt werden.

Gemäß Verfahrensschritt b) können die Verbindungen der allgemeinen Formel I aus Glycero-3(2)-phosphorsäureestern der allgemeinen Formel VII durch enzymatisch katalysierte Umesterung mit Hilfe von Phospholipase D nach an sich bekannten Methoden gewonnen werden.

Einige Phopsholipide sind bereits mit Hilfe von Phospholipase D hergestellt worden (H. Eibl et. al., Methods in Enzymology, 72, 1981, 632—639), wobei teilweise nicht natürlich vorkommende Glycerophosphorsäurealkylester und Analoge als Substrate in die Reaktion eingesetzt wurden. Bei der enzymatischen Synthese von Phosphatidylserinen aus natürlichen Phophatidylcholinen (P. Comfurius et. al. Biochim. Biophys. Acta 488, 1977, 36—42) wurden bisher nur bescheidene Ausbeuten an Phosphatidylserinen erhalten und vornehmlich Hydrolyse unter Bildung von Phosphatidsäuren beobachtet. Andere als natürliche Substrate sind bisher zur Herstellung von Phosphatidylserinen durch Umesterung mittels Phospholipase D nicht eingesetzt worden.

Im wesentlichen erfolgt die Herstellung der neuen Glycero-3(2)-phospho-L-serinderivate der allgemeinen Formel I dadurch, daß man eine Verbindung der allgemeinen Formel VII in wäßriger Lösung oder Suspension unter Zusatz von organischen Lösungsmitteln als Lösungsvermittler, z.B. Ether und/oder Chloroform und eines Puffers, z.B. Natriumacetat- oder Tris-Puffer, bei einem pH-Wert von 4.8 bis 8 in Gegenwart eines Calciumsalzes (Molarität vorzugsweise 0.01 bis 0.1 Mol/Liter) mit L-Serin in Anwesenheit von Phospholipase D bei Temperaturen zwischen 10 und 50°C reagieren läßt. Ein besonderer Vorteil des Verfahrens liegt darin, daß man ungeschütztes Serin zur Umsetzung verwenden kann.

Nach erfolgtem Umsatz, der beispielsweise dünnschichtchromatographisch verfolgt werden kann, wird das Enzym inaktiviert, vorteilhafterweise durch Zusatz von 0.1 M Ethylendiamintetraessigsäure Lösung, und anschließend das entstandene Glycero-3(2)-phospho-L-serinderivat der allgemeinen Formel I auf üblichen Weg, z.B. mit Hilfe chromatographischer Methoden, wie Dünnschicht-, Säulen- oder Hochdruckflüssigkeitschromatographie, isoliert und gereinigt.

Wie eingangs erwähnt, werden von der allgemeinen Formel I alle möglichen chiralen und diastereomeren Formen der erfindungsgemäßen Verbindungen umfaßt. Sowohl nach Verfahrensvariante a) als auch nach Verfahrensvariante b) können in Abhängigkeit vond en sterischen Verhältnissen in den Ausgangsmaterialien der Formel IV bzw. der Formel VII entweder chirale oder diastereomere Endprodukte erhalten werden. Verwendet man einer der beiden beschriebenen Verfahrensvarianten chirale Ausgangsmaterialien oder solche die über kein Chiralitätszentrum verfügen, erhält man chirale Formen von Verbindungen der allgemeinen Formel I. Setzt man hingegen racemische Ausgangsmaterialien der Formel IV bzw. der Formel VII ein, erhält man bei der erfindungsgemäßen Umsetzung mit einem geschützten L-Serinderivat der Formel VI bzw. mit L-Serin selbst Diastereomerengemische der Verbindungen der allgemeinen Formel I.

Die nach Verfahrensvariante a) oder b) erhaltenen Verbindungen der allgemeinen Formel I oder pharmazeutisch nicht annehmbare Salze davon können gemäß Verfahrensschritt c) auf übliche Weise mit anorganischen oder organischen Basen in ihre pharmazeutisch annehmbaren Salze übergeführt werden. Die Salzbildung kann beispielsweise durchgeführt werden, indem man die genannten Verbindungen der Formel I in einem geeigneten organischen Lösungsmittel, z.B. einem niederen aliphatischen Alkohol, löst, eine äquivalente Menge der gewünschten Base zusetzt, für eine gute Durchmischung sorgt und nach beendeter Salzbildung das Lösungsmittel im Vakuum abdestilliert.

Pharmazeutisch annehmbare Salze sind z.B. Metallsalze, insbesondere Alkalimetall- und Erdalkalimetallsalze, wie Natrium-, Kalium-, Magnesium- oder Calciumsalze. Andere pharmazeutisch annehmbare Salze sind beispielsweise auch Ammoniumsalze, die sich von Ammoniak oder organischen Aminen, z.B. Mono- Di- oder Tri-nieder-(alkyl, cycloalkyl oder hydroxyalkyl)-aminen, Niederalkylendiaminen oder heterocyclischen Basen, z.B. Methylamin, Diethylamin, Triethylamin, Dicyclohexylamin, Triethanolamin, Ethylendiamin, Pyridin, Piperidin, Piperazin, Morpholin und dergleichen ableiten.

Pharmazeutisch nicht annehmbare Salze von Verbindungen der allgemeinen Formel I können durch übliches Umsalzen in pharmazeutisch annehmbare Salze umgewandelt werden, wobei das pharmazeutisch nicht verwendbare Kation durch ein pharmazeutisch vewrendbares Kation ersetzt wird. Alternativ dazu kann man ein pharmazeutisch nicht verwendbares Salz auch neutralisieren und danach die so erhaltene freie Säure mit einer Base umsetzten, welche ein pharmazeutisch annehmbares Salz liefert.

Die für die Verfahrensvariante a) als Augangsmaterial verwendeten Glycero-3(2)-phosphorsäurederivate der allgemeinen Formel IV sind entweder bekannt oder können nach an sich bekannten Methoden (z.B. H. Brachwitz et al., Chem. Phys. Lipids 31, 1982, 33—52, A. Hermetter et al.,

Chem. Phys. Lipids, 30, 1982, 35—45), insbesondere nach den näheren Angaben in den Ausführungsbeispielen, hergestellt werden.

Die für die Verfahrensvariante b) als Ausgangsmaterial verwendeten Glycero-3(2)-phosphorsäureester der allgemeinen Formel VII sind ebenfalls entweder bekannt oder können nach an sich bekannten Methoden (DDR-Patent-Nr. 222 594 und Nr. 222 595) erhalten werden.

Eine für die enzymatische Umsetzung geeignete Phospholipase D kann in Anlehnung an bereits bekannte Methoden in einfacher Weise aus Weißkohl erhalten werden, indem man diesen homogenisiert, das Homogenisat filtriert und die wäßrige Phase 45 Minuten bei 25 000 g zentrifugiert. Der klare Überstand wird mit 2 Volumen Aceton versetzt. Vom entstandenen Niederschlag wird unter Benutzung einer Tauchfritte dekantiert und der Rückstand 20 Minuten bei 13000 g und 5°C zentrifugiert. Das aceton-feuchte enzymhältige Präparat wird über Phosphorpentoxid im Vakuum getrocknet.

Die Verbindungen der allgemeinen Formel I und Salze davon sind biologisch hochaktiv und besitzen insbesondere eine ausgeprägte Antitumorwirksamkeit. Diese wertvollen pharmakologischen Eigenschaften können in vitro und in vivo unter Verwendung von Standardmethoden nachgewiesen werden, beispielsweise indem die Hemmung der Proliferation von Ehrlich-Ascites-Tumorzellen in vitro unter Einwirkung von Glycero-3(2)-phospho-L-serinderivaten der allgemeinen Formel I bestimmt wird.

In diesem Test (Tabelle I) bewirken die erfindungsgemäßen Verbindungen, beispielsweise

1-O-Hexadecyl-2-chlor-2-desoxy-glycero-3-phospho-L-serin (Verbindung Nr. 1)
1-O-Hexadecyl-2-fluor-2-desoxy-glycero-3-phospho-L-serin (Verbindung Nr. 2)
1-Chlor-1-desoxy-3-O-hexadecyl-glycero-2-phospho-L-serin (Verbindung Nr. 3)
1-O-(2,2,2-Trifluorethy)-2-O-hexadecyl-glycero-3-phospho-L-serin (Verbindung Nr. 4),

schon in sehr geringen Konzentrationen eine signifikante Hemmung des Zellwachstums von Ehrlich-Ascites-Tumorzellen.

TABELLE I

Hemmung der Proliferation (%) von Ehrlich-Ascites-Tumorzellen in vitro durch Verbindungen der allgemeinen Formel I in Abhängigkeit von der Konzentration

| | Konzentration (µM) | | | | | |
|---|---|---|---|---|---|---|
| | 100 | 20 | 10 | 5 | 2 | 1 |
| Verbindung Nr. | | | % Hemmung | | | |
| 1 | 95 | 80 | 55 | 30 | 10 | 16 |
| 2 | 95 | 82 | 51 | 18 | 13 | 10 |
| 3 | 93 | 81 | 68 | 38 | 17 | 15 |
| 4 | 100 | 74 | 36 | 10 | | |
| V | 100 | 62 | 23 | 4 | 0 | 0 |

V=Vergleichssubstanz: 1-O-Octadecyl-2-O-methyl-glycero-3-phosphocholin.

Die in vitro-Wirksamkeit der erfindungsgemäßen Verbindungen ist qualitativ vergleichbar mit dem cytostatisch wirksamen 1-O-Octadecyl-2-O-methyl-glycero-3-phosphocholin, das bereits klinische Anwendung in der Krebstherapie gefunden hat (P. G. Munder et al. in: "Augmentig Agents in Cancer Therapy", S. 441—458, Raven Press, New York, 1981; W. E. Berdel et al. Cancer, 50, 1982, 2011—2015). Gegenüber der Vergleichssubstanz haben die erfindungsgemäßen Verbindungen den Vorteil, daß eine signifikante Antitumorwirkung bereits bei wesentlich niedrigeren Konzentrationen eintritt, so daß zur Erzielung desselben cytostatischen Effekts erheblich geringere Dosiseinheiten verabreicht werden müssen. Aufgrund dieser Eigenschaften lassen die erfindungsgemäßen Verbindungen eine vorteilhaftere Verwendung in der Haumanmedizin zur Behandlung und Prophylaxe von Tumorerkrankungen erwarten.

Insgesamt ist die ausgeprägte Antitumorwirkung der erfindungsgemäßen Glycero-3(2)-phospho-L-serinderivate überraschend, da bisher angenommen wurde (vgl. D. R. Hoffmann et al., Research Commun in Chem. Pathology and Pharmacology, 44, 1984, 239—306), daß die Antitumorwirkung von Alkylphospholipid-Analogen auf Verbindungen, die eine Phosphocholingruppierung besitzen, beschränkt ist.

Die Verbindungen der allgemeinen Formel I können als Heilmittel, z.B. in Form von pharmazeutischen Präparaten Verwendung finden, welche sie in Mischung mit einem für die enterale oder parenterale Applikation geeigneten pharmazeutischen, organischen oder anorganischen inerten Hilfs- und/oder Trägermaterial, wie z.B. pharmazeutisch unbedenklichen Lösungsmitteln, Gelatine, Gummi arabicum, Milchzucker, Stärke, Magnesiumstearat, Talk, pflanzliche Öle, Polyalkylenglykole, Vaseline und dergleichen, enthalten.

Die pharmazeutischen Präparate können in fester Form, beispielsweise als Tabletten, Dragees, Suppositorien, Kapseln und dergleichen oder in flüssiger Form, beispielsweise als Lösungen, Suspensionen oder Emulsionen, vorliegen. Gegebenenfalls sind sie sterilisiert und enthalten Hilfstoffe, wie Konservierungs-, Stabilisierungs- oder Emulgiermittel, Salze zur Veränderung des osmotischen Druckes und dergleichen.

Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen zusammen mit den oben angegebenen Hilfs- und/oder Trägerstoffen zu Kombinationspräparaten formuliert werden.

Die folgenden Beispiele erläutern die Erfindung näher:

Beispiel 1:
1-O-Hexadecyl-2-chlor-2-desoxy-glycero-3-phospho-L-serin

1-O-Hexadecyl-2-chlor-2-desoxy-glycero-3-phosphorsäure:

335 mg (1 mMol) 1-O-Hexadecyl-2-chloro-2-desoxy-glycerol werden in 10 ml trockenem Tetrahydrofuran gelöst, 0.6 ml Pyridin zugefügt, die so erhaltene Lösung zu einer Lösung von 0.35 ml (3,755 mMol) Phosphoroxychlorid in 3.5 ml wasserfreiem Tetrahydrofuran bei 0°C unter Rühren zugetropft und weitere 3 Stunden bei 0°C gerührt. Dann werden 16 ml einer 10 %-igen Natriumbicarbonatsuspension zugefügt, 15 Minuten gerührt, mit verdünnter Salzsäure auf einen pH von 7 gestellt und mehrmals mit Ether/Chloroform extrahiert. Die Extrakte werden im Vakuum eingedampft, man erhält so 390 mg (92% der Theorie) Rohprodukt, das für wietere Umsetzungen ausreichend rein ist.

Rf: 0.15 (Kieselgel 60, Alufolie Merck; $CHCl_3/CH_3OH/25$ %-iger $NH_3$=50:25:6, v/v/v).

1-O-Hexadecyl-2-chlor-2-desoxy-glycero-3-phospho-N-tert.butoxycarbonyl-L-serinbenzhydrylester:

225 mg (0.54 mMol) 1-O-Hexadecyl-2-chloro-2-desoxy-glycero-3-phosphorsäure, 0.3 g (0.81 mMol) N-tert.Butoxycarbonyl-L-serinbenzhydrylester und 0.657 g (2.35 mMol), 2,4,6-Triisopropylbenzolsulfochlorid werden in 13 ml wasserfreiem Pyridin 36 Stunden bei Raumtemperatur gerührt. Es werden einige Tropfen Wasser zugegeben, im Vakuum eingedampft, mehrmals mit Toluol nachdestilliert, der Rückstand in Ether aufgenommen, filtriert und eingedampft.

Das so erhaltene Rohprodukt wird an Kieselgel (35 g, KG 60, Merck 40—63 µm) adsorbiert und aufeinanderfolgend mit 50 ml Chloroform und Chloroform/Methanol (9:1) eluiert. Es werden Fraktionen zu je 15 ml gewonnen. Die Fraktionen 6—12 werden vereinigt und eingedampft. Man erhält so 161 mg (35% der Theorie) reines Produkt.

Rf: 0.72 (Kieselgel 60, Alufolie Merck; $CHCl_3/CH_3OH/25$ %-iger $NH_3$=65:35:5, v/v/v).

1-O-Hexadecyl-2-chlor-2-desoxy-glycero-3-phospho-L-serin:

161 mg (0.21 mMol) 1-O-Hexadecyl-2-chlor-2-desoxy-glycero-3-phospho-N-tert.butoxycarbonyl-L-serin-benzhydrylester werden in 30 ml trockenem Chloroform gelöst und unter Rühren bei 0°C 15 Minuten trockenes HCl-Gas durchgeleitet. Anschließend wird 1 Stunde trockener Stickstoff eingeleitet. Die Lösung wird dann mit verdünntem wäßrigem Ammoniak und Wasser gewaschen und eingeengt.

Das so erhaltene Rohprodukt wird über Kieselgel (10 g KG 60, Merck 40—63 µm; Elutionsmittel: $CHCl_3/CH_3OH$, 2:1 v/v mit ansteigendem Methanolgradierten) gereinigt. Nach Vereinigung der im Dünnschichtchromatogramm einheitlichen Fraktionen und Abdampfen des Lösungsmittels erhält man 38 mg (36% der Theorie) reines Produkt.

Rf: 0.13 (Kieselgel 60, Alufolie Merck; $CHCl_3/CH_3OH/H_2O$=50:25:4, v/v/v):

$C_{22}H_{45}ClPNO_7$ (502, 02) Ber. C 52.63 H 9.04 N 2.79
Gef. C 51.76 H 8.82 N 2.60

Analog der in Beispiel 1 angegebenen Vorschrift werden in den Beispielen 2—9 hergestellt:

Beispiel 2:
1-O-Hexadecyl-2-fluor-2-desoxy-glycero-3-phospho-L-serin

1-O-Hexadecyl-2-fluoro-2-desoxy-glycero-3-phosphorsäure:
aus 334 mg (1.05 mMol) 1-O-Hexadecyl-2-fluor-2-desoxy-glycerol
Ausbeute: 320 mg (76% der Theorie).
Rf: 0.15 (Kieselgel 60, Alufolie Merck; $CHCl_3/CH_3OH/25$ %-iger $NH_3$=50:25:6, v/v/v).

1-O-Hexadecyl-2-fluor-2-desoxy-glycero-3-phospho-N-tert.butoxycarbonyl-L-serin-benzhydrylester:
aus 184 mg (0.46 mMol) 1-O-Hexadecyl-2-fluor-2-desoxy-glycero-3-phosphorsäure.
Ausbeute 190 mg (54% der Theorie) reines Produkt.
Rf: 0.75 (Kieselgel 60, Alufolie Merck; $CHCl_3/CH_3OH/25$ %-iger $NH_3$=65:35:5, v/v/v).

1-O-Hexadecyl-2-fluor-2-desoxy-glycero-3-phospho-L-serin:
aus 190 mg (0.25 mMol) 1-O-Hexadecyl-2-fluor-2-desoxy-glycero-3-phospho-N-tert.butoxycarbonyl-L-serin-benzhydrylester.
Ausbeute: 88 mg (72% der Theorie) reines Produkt.
Rf: 0.12 (Kieselgel 60, Alufolie Merck; $CHCl_3/CH_3OH/H_2O$=50:25:4, v/v/v).

Beispiel 3:
1-Chloro-1-desoxy-3-O-hexadecyl-glycero-2-phospho-L-serin

1-Chlor-1-desoxy-3-O-hexadecyl-glycerol-2-phosphorsäure:
aus 350 mg (1.05 mMol) 1-Chlor-1-desoxy-3-O-hexadecyl-glycerol.
Ausbeute: 322 mg (74% der Theorie).
Rf: 0.18 (Kieselgel 60, Alufolie Merck; $CHCl_3/CH_3OH$/25 %-iger $NH_3$=50:25:6, v/v/v).

1-Chlor-1-desoxy-3-O-hexadecyl-glycero-2-phospho-N-tert.butoxycarbonyl-L-serin-benzhydrylester:
aus 322 mg (0.77 mMol) 1-Chlor-1-desoxy-3-O-hexadecyl-glycero-2-phosphorsäure.
Ausbeute: 318 mg (53% der Theorie) reines Produkt.
Rf: 0.80 (Kieselgel 60, Alufolie Merck; $CHCl_3/CH_3OH$/25 %-iger $NH_3$=65:35:5, v/v/v).

1-Chlor-1-desoxy-3-O-hexadecyl-glycero-2-phospho-L-serin:
aus 308 mg (0.40 mMol) 1-Chlor-1-desoxy-3-O-hexadecyl-glycero-2-phospho-N-tert.butoxycarbonyl-L-serinbenzhydrylester.
Ausbeute: 87 mg (43% der Theorie) reines Produkt.
Rf: 0.13 (Kieselgel 60, Alufolie Merck; $CHCl_3/CH_3OH/H_2O$=50:25:4, v/v/v).

Beispiel 4:
1-Chlor-1-desoxy-3-O-octadecyl-glycero-2-phospho-L-serin

1-Chloro-1-desoxy-3-O-octadecyl-glycero-2-phosphorsäure:
aus 417 mg (1.15 mMol) 1-Chlor-1-desoxy-3-O-octadecyl-glycerol.
Ausbeute: 450 mg (89% der Theorie).
Rf: 0.18 (Kieselgel 60, Alufolie Merck; $CHCl_3/CH_3OH$/25 %-iger $NH_3$=50:25:6, v/v/v).

1-Chloro-1-desoxy-3-O-octadecyl-glycero-2-phospho-N-tert.butoxycarbonyl-L-serinbenzhydrylester:
aus 320 mg (0.72 mMol) 1-Chlor-1-desoxy-3-O-octadecyl-glycero-2-phopshorsäure.
Ausbeute: 258 mg (45% der Theorie) reines Produkt.
Rf: 0.80 (Kieselgel 60, Alufolie Merck; $CHCl_3/CH_3OH$/25 %-iger $NH_3$=65:35:5, v/v/v).

1-Chlor-1-desoxy-3-O-octadecyl-glycero-2-phospho-L-serin:
aus 258 mg (0.32 mMol) 1-Chlor-1-desoxy-3-O-octadecyl-glycero-2-phospho-N-tert.butoxycarbonyl-L-serinbenzhydrylester.
Ausbeute: 61 mg (36% der Theorie) reines Produkt.
Rf: 0.13 (Kieselgel 60, Alufolie Merck; $CHCl_3/CH_3OH/H_2O$=50:25:4, v/v/v).

Beispiel 5:
1-O-Octadecyl-2-fluor-2-desoxy-glycero-3-phospho-L-serin

1-O-Octadecyl-2-fluor-2-desoxy-glycero-3-phosphorsäure:
aus 346 mg (1 mMol) 1-O-Octadecyl-2-fluor-2-desoxy-glycerol.
Ausbeute: 324 mg (76% der Theorie).
Rf: 0.15 (Kieselgel 60, Alufolie Merck; $CHCl_3/CH_3OH$/25 %-iger $NH_3$=50:25:6, v/v/v).

1-O-Octadecyl-2-fluor-2-desoxy-glycero-3-phospho-N-tert.butyloxycarbonyl-L-serinbenzhydrylester:
aus 307 mg (0.72 mMol) 1-O-Octadecyl-2-fluor-2-desoxy-glycero-3-phosphorsäure:
Ausbeute: 281 mg (50% der Theorie) reines Produkt.
Rf: 0.75 (Kieselgel 60, Alufolie Merck; $CHCl_3/CH_3OH$/25 %-iger $NH_3$=65:35:5, v/v/v).

1-O-Octadecyl-2-fluor-2-desoxy-glycero-3-phospho-L-serin:
aus 281 mg (0.36 mMol) 1-O-Octadecyl-2-fluor-2-desoxy-glycero-3-phospho-N-tert.butoxycarbonyl-L-serinbenzhydrylester.
Ausbeute: 54 mg (29% der Theorie) reines Produkt.
Rf: 0.13 (Kieselgel 60, Alufolie Merck; $CHCl_3$, $CH_3OH/H_2O$=50:25:4, v/v/v).

Beispiel 6:
1-O-Octadecyl-2-chlor-2-desoxy-glycero-3-phospho-L-serin:

1-O-Octadecyl-2-chlor-2-desoxy-glycero-3-phosphorsäure:
aus 544 mg (1,5 mMol) 1-O-Octadecyl-2-chlor-2-desoxy-glycerol.
Ausbeute: 597 mg (90% der Theorie).
Rf: 0.16 (Kieselgel 60, Alufolie Merck; $CHCl_3/CH_3OH$/25 %-iger $NH_3$=50:25:6, v/v/v).

1-O-Octadecyl-2-chlor-2-desoxy-glycero-3-phospho-N-tert.butoxycarbonyl-L-serinbenzhydrylester:
aus 443 mg (1 mMol) 1-O-Octadecyl-2-chlor-2-desoxy-glycero-3-phosphorsäure.
Ausbeute: 382 mg (48% der Theorie) reines Produkt.
Rf: 0.80 (Kieselgel 60, Alufolie Merck; $CHCl_3/CH_3OH$/25 %-iger $NH_3$=65:35:5, v/v/v).

1-O-Octadecyl-2-chlor-2-desoxy-glycero-3-phospho-L-serin:
aus 382 mg (0.48 mMol) 1-O-Octadecyl-2-chlor-2-desoxy-glycero-3-phospho-N-tert.butoxycarbonyl-L-serinbenzhydrylester.
Ausbeute: 102 mg (40% der Theorie) reines Produkt.
Rf: 0.13 (Kieselgel 60, Alufolie Merck; $CHCl_3/CH_3OH/H_2O$=50:25:4, v/v/v).

Beispiel 7:

1-O-Tetradecyl-2-chlor-2-desoxy-glycero-3-phospho-L-serin

1-O-Tetradecyl-2-chlor-2-desoxy-glycero-3-phosphorsäure:
aus 368 mg (1.2 mMol) 1-O-Tetradecyl-2-chlor-2-desoxy-glycerol.
Ausbeute: 445 mg (96% der Theorie).
Rf: 0.14 (Kieselgel 60, Alufolie Merck; $CHCl_3/CH_3OH/25$ %-iger $NH_3=50:25:6$, v/v/v).

1-O-Tetradecyl-2-chlor-2-desoxy-glycero-3-phospho-N-tert.butoxycarbonyl-L-serinbenzhydrylester:
aus 425 mg (1.1 mMol) 1-O-Tetradecyl-2-chlor-2-desoxy-glycero-3-phosphorsäure.
Ausbeute: 423 mg (52% der Theorie) reines Produkt.
Rf: 0.72 (Kieselgel 60, Alufolie Merck; $CHCl_3/CH_3OH/25$ %-iger $NH_3=65:35:5$, v/v/v).

1-O-Tetradecyl-2-chlor-2-desoxy-glycero-3-phospho-L-serin:
aus 192 mg (0.26 mMol) 1-O-Tetradecyl-2-chlor-2-desoxy-glycero-3-phospho-N-tert.butoxycarbonyl-L-serinbenzhydrylester.
Ausbeute: 39.5 mg (32% der Theorie) reines Produkt.
Rf: 0.13 (Kieselgel 60, Alufolie Merck; $CHCl_3/CH_3OH/H_2O=50:25:4$, v/v/v).

Beispiel 8:

1-O-(1-Methyl-heptadecyl)-2-chlor-2-desoxy-glycero-3-phospho-L-serin

1-O-(1-Methyl-heptadecyl)-2-chlor-2-desoxy-glycero-3-phosphorsäure:
aus 363 mg (1 mMol) 1-O-(1-Methyl-heptadecyl)-2-chlor-2-desoxy-glycerol.
Ausbeute: 372 mg (84% der Theorie).
Rf: 0.15 (Kieselgel 60, Alufolie Merck; $CHCl_3/CH_3OH/25$ %-iger $NH_3=50:25:6$, v/v/v).

1-O-(1-Methyl-heptadecyl)-2-chlor-2-desoxy-glycero-3-phospho-N-tert.butoxycarbonyl-L-serinbenzhydrylester:
aus 350 mg (0.79 mMol) 1-O-(1-Methyl-heptadecyl)-2-chlor-2-desoxy-glycero-3-phosphorsäure.
Ausbeute: 371 mg (59% der Theorie) reines Produkt.
Rf: 0.80 (Kieselgel 60, Alufolie Merck; $CHCl_3/CH_3OH/25$ %-iger $NH_3=65:35:5$, v/v/v).

1-O-(1-Methyl-heptadecyl)-2-chlor-2-desoxy-glycero-3-phospho-L-serin:
aus 370 mg (0.59 mMol) 1-O-(1-Methyl-heptadecyl)-2-chlor-2-desoxy-glycero-3-phospho-N-tert.butoxycarbonyl-L-serinbenzhydrylester.
Ausbeute: 122 mg (39% der Theorie) reines Produkt.
Rf: 0.14 (Kieselgel 60, Alufolie Merck; $CHCl_3/CH_3OH/H_2O=50:25:4$, v/v/v).

Beispiel 9:

1-O-Pentyl-2-chlor-2-desoxy-glycero-3-phospho-L-serin

1-O-Pentyl-2-chlor-2-desoxy-glycero-3-phosphorsäure:
aus 271 mg (1.5 mMol) 1-O-Pentyl-2-chlor-2-desoxy-glycerol.
Ausbeute: 250 mg (64% der Theorie).
Rf: 0.10 (Kieselgel 60, Alufolie Merck; $CHCl_3/CH_3OH/25$ %-iger $NH_3=50:25:6$, v/v/v).

1-O-Pentyl-2-chlor-2-desoxy-glycero-3-phospho-N-tert.butoxycarbonyl-L-serinbenzhydrylester:
aus 208 mg (0.8 mMol) 1-O-Pentyl-2-chlor-2-desoxy-glycero-3-phosphorsäure.
Ausbeute: 407 mg (83% der Theorie) reines Produkt.
Rf: 0.70 (Kieselgel 60, Alufolie Merck; $CHCl_3/CH_3OH/25$ %-iger $NH_3=65:35:5$, v/v/v).

1-O-Pentyl-2-chlor-2-desoxy-glycero-3-phospho-L-serin:
aus 406 mg (0.66 mMol) 1-O-Pentyl-2-chlor-2-desoxy-glycero-3-phospho-N-tert.butoxy-L-serinbenzhydrylester.
Ausbeute: 64 mg (28% der Theorie) reines Produkt.
Rf: 0.11 (Kieselgel 60, Alufolie Merck; $CHCl_3/CH_3OH/H_2O=50:25:4$, v/v/v).

Beispiel 10:

1-O-Hexadecyl-2-desoxy-glycero-3-phospho-L-serin

Eine Mischung von 1 g L-Serin, 1.9 ml 0.1 M Acetatpuffer (pH 5.6) mit 0.1 M $CaCl_2$, 40 mg 1-O-Hexadecyl-2-desoxy-glycero-phosphorsäureethylester, 2 ml Ether/Chloroform (9:1, v/v) und 100 mg eines aus etwa 500 g Weißkohl gewonnenen Phospholipase-D-Präparates wird 40 Stunden bei 40°C intensiv gerührt.

Nach dem Abkühlen auf Raumtemperatur werden 4.35 ml 0.1 M Ethylendiamintetraessigsäure hinzugegeben. Die organischen Lösungsmittel werden durch Einleiten von Stickstoff entfernt. Die Mischung wird mit dem 4.3-fachen Volumen Chloroform/Methanol (5:8, v/v) 30 Minuten gerührt und das sich hierbei ausscheidende nicht umgesetzte Serin durch Absaugen entfernt.

Das Filtrat wird mit 1 Volumen Wasser und 3.7 Volumen Chloroform 10 Minuten gerührt, die organische Phase abgetrennt und eingeengt. Der erhaltene Rückstand wird an 20 g Carboxymethylcellulose (Servacel CM 52) säulenchromatographisch getrennt, wobei die Elution nacheinander mit 75 ml Chloroform (Fraktion 1), je 500 ml Chloroform/Methanol (9:1, 8:2, 7:3, 1:1, v/v), Fraktionen 2—5, vorgenommen wird. Das Endprodukt wird aus Fraktion 5 in reiner Form erhalten.

Ausbeute: 15 mg (33% der Theorie) reines Produkt.

Rf: 0.13 (Merck, Kiesegel 60, Fertigplatte; $CHCl_3/CH_3OH/H_2O=50:25:4$, v/v/v).
Analog zu dieser Vorschrift wird in den Beispielen 11—21 hergestellt.

Beispiel 11:
1-O-Hexadecyl-2-O-(2,2,2-trifluorethyl)-glycero-3-phospho-L-serin
aus 40 mg 1-O-Hexadecyl-2-O-(2,2,2-trifluorethyl)-glycero-3-phospho-cholin.
Ausbeute: 12 mg (30% der Theorie) reines Produkt.
Rf: 0.13 (Merck Kieselgel 60, Fertigplatte; $CHCl_3/CH_3OH/H_2O=50:25:4$, v/v/v).

Beispiel 12:
1-O-Hexadecyl-2-O-(2,2,2-trifluorethyl)-glycero-3-phospho-L-serin
aus 40 mg 1-O-Hexadecyl-2-O-(2,2,2-trifluorethyl)-glycero-3-phosphorsäure-2-bromethylester).
Ausbeute: 13.5 mg (35% der Theorie) reines Produkt.
Rf: 0.13 (Merck Kieselgel 60, Fertigplatte; $CHCl_3/CH_3OH/H_2O=50:25:4$, v/v/v).

Beispiel 13:
1-Chlor-1-desoxy-2-O-hexadecyl-glycero-3-phospho-L-serin
aus 40 mg 1-Chlor-1-desoxy-2-O-hexadecyl-glycero-3-phosphocholin.
Ausbeute: 15 mg (37% der Theorie) reines Produkt.
Rf; 0.14 (Merck Kieselgel 60, Fertigplatte; $CHCl_3/CH_3OH/H_2O=50:25:4$, v/v/v).

Beispiel 14:
1-O-(2,2,2-Trifluorethyl)-2-O-hexadecyl-glycero-3-phospho-L-serin
aus 40 mg 1-O-(2,2,2-Trifluorethyl)-2-O-hexadecyl-glycero-3-phosphocholin.
Ausbeute: 18 mg (44% der Theorie) reines Produkt.
Rf: 0.13 (Merck Kieselgel 60, Fertigplatte; $CHCl_3/CH_3OH/H_2O=50:25:4$, v/v/v).

Beispiel 15:
1-O-Eicosanyl-2-chlor-2-desoxy-glycero-3-phospho-L-serin
aus 40 mg 1-O-Eicosanyl-2-chlor-2-desoxy-glycero-3-phosphorsäure-n-butylester.
Ausbeute: 14.5 mg (34% der Theorie) reines Produkt.
Rf: 0.15 (Merck Kiesegel 60, Fertigplatte; $CHCl_3/CH_3OH/H_2O=50:25:4$, v/v/v).

Beispiel 16:
1-O-Triacontyl-2-chlor-2-desoxy-glycero-3-phospho-L-serin
aus 60 mg 1-O-Triacontyl-2-chlor-2-desoxy-glycero-3-phosphorsäureethylester.
Ausbeute: 14 mg (33% der Theorie) reines Produkt.
Rf: 0.20 (Merck Kieselgel 60, Fertigplatte; $CHCl_3/CH_3OH/H_2O=50:25:4$, v/v/v).

Beispiel 17:
1-O-Octadecyl-2-O-(2,2,2-trifluorethyl)-glycero-3-phospho-L-serin
aus 40 mg 1-O-Octadecyl-2-O-(2,2,2-trifluorethyl)-glycero-3-phosphorsäure-2-bromethylester.
Ausbeute: 13.5 g (32% der Theorie) reines Produkt.
Rf: 0.14 (Merck Kieselgel 60, Fertigplatte; $CHCl_3/CH_3OH/H_2O=50:25:4$, v/v/v).

Beispiel 18:
1,2-Di-O-hexadecyl-glycero-3-phospho-L-serin
aus 40 mg 1,2-Di-O-hexadecyl-glycero-3-phosphocholin.
Ausbeute: 13 mg (32% der Theorie) reines Produkt.
Rf: 0.22 (Merck Kieselgel 60, Fertigplatte; $CHCl_3/CH_3OH/H_2O=50:25:4$, v/v/v).

Beispiel 19:
1-O-Eicosanyl-2-O-(2,2,2-trifluorethyl)-glycero-3-phospho-L-serin
aus 40 mg 1-O-Eicosanyl-2-O-(2,2,2-trifluorethyl)-glycero-3-phosphocholin.
Ausbeute: 16 mg (40% der Theorie) reines Produkt.
Rf: 0.14 (Merck Kieselgel 60, Fertigplatte; $CHCl_3/CH_3OH/H_2O=50:25:4$, v/v/v).

Beispiel 20:
1-Chlor-1-desoxy-3-O-(cis-9-octadecenyl)-glycero-2-phospho-L-serin
aus 40 mg 1-Chlor-1-desoxy-3-O-(cis-9-octadecenyl)-glycero-2-phosphorsäureethylester.
Ausbeute: 15.5 mg (34% der Theorie) reines Produkt.
Rf: 0.13 (Merck Kieselgel 60, Fertigplatte; $CHCl_3/CH_3OH/H_2O=50:25:4$, v/v/v).

Beispiel 21:
1-O-(2-Methoxy-octadecyl)-2-chlor-2-desoxy-glycero-3-phospho-L-serin
aus 50 mg 1-O-(2-Methoxy-octadecyl)-2-chlor-2-desoxy-glycero-3-phosphorsäureethylester.
Ausbeute: 16 mg (28% der Theorie) reines Produkt.
Rf: 0.15 (Merck Kieselgel 60, Fertigplatte; $CHCl_3/CH_3OH/H_2O=50:25:4$, v/v/v).

**Patentansprüche**

1. Glycero-3(2)-phospho-L-serinderivate der allgemeinen Formel

$$\begin{array}{l} CH_2—A \\ | \\ CH—B \\ | \\ CH_2—C \end{array} \qquad I$$

in der
A unsubstituiertes oder ein- oder mehrfach durch Halogen, Hydroxy, Alkoxy oder Cyano substituiertes ($C_5$—$C_{30}$)-Alkoxy oder unsubstituiertes oder ein- oder mehrfach durch Halogen, Hydroxy, Alkoxy oder Cyano substituiertes ($C_5$—$C_{30}$)-Alkenoxy, wobei eine Doppelbindung des Alkenoxyrestes nicht von dem an den Sauerstoff gebundenen C-Atom ausgeht, Halogen oder eine Gruppe der allgemeinen Formel

$$—O—(CH_2)_n—CF_3 \qquad II$$

worin
n für 0 oder eine ganze Zahl 1, 2 oder 3 steht, beduetet, einer der beiden Reste
B und C, der gleich wie A oder davon verschieden ist, eine der für A angegebenen Bedeutungen hat oder Wasserstoff bedeutet und der jeweils andere Rest die Phosphatidyl-L-serin-Gruppe der formel

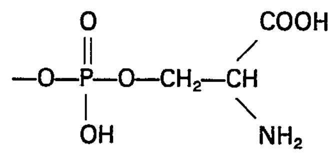
III

darstellt, mit der Maßgabe, daß mindestens ein Rest A, B oder C ($C_5$—$C_{30}$)-Alkoxy oder ($C_5$—$C_{30}$)-Alkenoxy bedeutet.

2. Verbindungen nach Anspruch 1, worin mindestens ein Rest A, B oder C ($C_{16}$—$C_{18}$)-Alkoxy oder ($C_{16}$—$C_{18}$)-Alkenoxy bedeutet.

3. Verbindungen nach Anspruch 1, worin ein Rest A, B oder C Chlor oder Fluor bedeutet.

4. Verbindungen nach einem der Ansprüche 1 bis 3, worin C die Phosphatidyl-L-serin-Gruppe der Formel III, einer der Reste A und B Alkoxy oder Alkenoxy und der jeweils andere Halogen bedeutet.

5. Verbindungen nach einem der Ansprüche 1 bis 3, worin B die Phosphatidyl-L-serin-Gruppe der Formel III, einer der Reste A und C Alkoxy oder Alkenoxy und der jeweils andere Halogen bedeutet.

6. Verbindungen nach einem der Ansprüche 1 oder 2, worin A Alkoxy oder Alkenoxy, B Wasserstoff oder Alkoxy und C die Phosphatidyl-L-serin-Gruppe der Formel III bedeutet.

7. Verbindungen nach einem der Ansprüche 1 oder 2, worin C die Phosphatidyl-L-serin-Gruppe der Formel III, einer der Reste A und B Alkoxy oder Alkenoxy und der jeweils andere einen endständig fluorierten Alkoxyrest der Formel II bedeutet.

8. Verbindungen nach einem der Ansprüche 1 bis 7, dadurch gekennzeichnet, daß sie als Diastereomere vorliegen.

9. Verfahren zur Herstellung von Glycero 3(2)-phospho-L-serin-derivaten der in Anspruch 1 definierten allgemeinen Formel I, dadurch gekennzeichnet, daß man
a) ein Glycero-3(2)-phosphorsäurederivat der allgemeinen Formel

$$\begin{array}{l} CH_2—A \\ | \\ CH—D \\ | \\ CH_2—E \end{array} \qquad IV$$

in der
A wie in Formel I definiert ist, einer der beiden Reste
D und E eine der in Formel I für A angegebenen Bedeutungen hat oder Wasserstoff ist und der jeweils andere Rest eine Gruppe der allgemeinen Formel

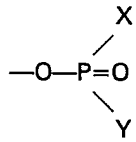

V

worin

X und Y entweder gleich sind und Hydroxy oder Halogen bedeuten oder Y für niederes Alkoxy oder Aryloxy steht, darstellt, mit der Maßgabe, daß mindestens ein Rest A, D oder E ($C_5$—$C_{30}$)-Alkoxy oder ($C_5$—$C_{30}$)-Alkenoxy bedeutet, oder dessen Salze mit einem geschützten L-Serinderivat der allgemeinen Formel

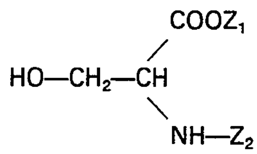

VI

in der

$Z_1$ eine Carboxyl- und

$Z_2$ eine Aminoschutzgruppe bedeutet, vorzugsweise in Anwesenheit eines Kondensationsmittels, umsetzt, anschließend in beliebiger Reihenfolge oder gleichzeitig die Schutzgruppen $Z_1$ und $Z_2$ abspaltet und gegebenenfalls entstandene Glycerophosphorsäureester oder-halogenide verseift, oder

b) einem Glycero-3(2)-phosphorsäureester der allgemeinen Formel

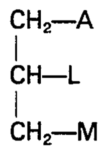

VII

in der

A wie in Formel I definiert ist, einer der beiden Reste

L und M eine der in Formel I für A angegebenen Bedeutungen hat oder Wasserstoff ist und der jeweils andere Rest eine Gruppe der allgemeinen Formel

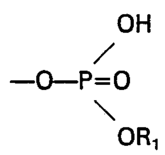

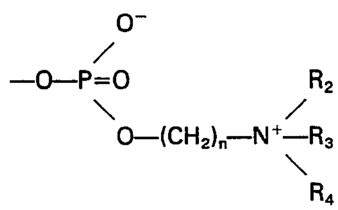

VIIIa oder VIIIb

worin

$R_1$ gegebenenfalls durch Hydroxy oder Halogen substituiertes ($C_1$—$C_6$)-Alkyl,

$R_2$, $R_3$ und $R_4$, die gleich oder verschieden sind, unabhängig voneinander Wasserstoff oder Methyl und

n eine ganze Zahl von 1—6 bedeuten, darstellt, mit der Maßgabe, daß mindestens ein Rest A, L oder M ($C_5$—$C_{30}$)-Alkoxy oder ($C_5$—$C_{30}$)-Alkenoxy bedeutet, mit L-Serin in Gegenwart von Phospholipase D zur Umsetzung bringt und die entstandenen Verbindungen der allgemeinen Formel I oder deren Salze isoliert, und

c) erwünschtenfalls eine nach Verfahrensvariante a) oder b) erhaltene Verbindung der allgemeinen Formel I oder ein pharmazeutisch nicht annehmbares Salz davon in ein pharmazeutisch annehmbares Salz überführt.

10. Pharmazeutische Präparate, enthaltend Verbindungen der allgemeinen Formel I nach Anspruch 1 oder ein Salz davon in Kombination mit üblichen galenischen Hilfs- und/oder Trägerstoffen.

**Revendications**

1. Composés glycéro-3(2)-phospho-L-sériniques de formule générale

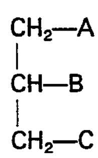

I

dans laquelle:

A représente:

un groupe alcoxy de 5 à 30 atomes de carbone non substitué ou substitué une ou plusieurs fois par un halogène, un groupe hydroxy, alcoxy ou cyano,

ou bien un groupe alcèneoxy de 5 à 30 atomes de carbone non substitué ou substitué une ou plusieurs fois par un halogène, un groupe hydroxy, alcoxy ou cyano et dans lequel la double liaison du reste alcèneoxy n'est pas sur l'atome de carbone lié à l'oxygène,

les halogènes,

ou un groupe de formule générale:

$$-O-(CH_2)_n-CF_3 \qquad II$$

où n est égal a 0, 1, 2 ou 3,

un des restes B et C identiques ou différents de A, ou bien l'un identique à A ou signifie l'hydrogène et l'autre signifie un groupe L-sérine phosphatidyle de formule

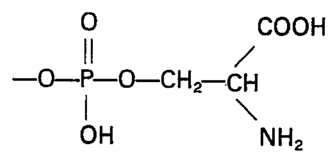

III

sous réserve qu'au moins un reste A, B ou C signifie un groupe alkoxy ou alcèneoxy de 5 à 30 atomes de carbone.

2. Composés selon la revendication 1, dans lesquels un reste A, B ou C signifie un groupe alcoxy ou alcèneoxy de 16 à 18 atomes de carbone.

3. Composés selon la revendication 1, dans lesquels un reste A, B ou C signifie le chlore ou le fluor.

4. Composés selon l'une des revendications 1 à 3, dans lesquels C signifie le groupe phosphatidyl-L-sérine de formule III, un des restes A et B un groupe alcoxy ou alcèneoxy et l'autre les halogènes.

5. Composés selon l'une des revendications 1 à 3, dans lesquels B signifie le groupe phosphatidyl-L-sérine de formule III, un des restes A et C un groupe alcoxy ou alcèneoxy et l'autre les halogènes.

6. Composés selon l'une des revendications 1 ou 2, dans lesquels A est un groupe alcoxy ou alcèneoxy, B l'hydrogène ou alcoxy et C le groupe phosphatidyl-L-sérine de formule III.

7. Composés selon l'une des revendications 1 ou 2, dans lesquels C signifie un groupe phosphatidyl-L-sérine de formule III, un des restes A et B un groupe alcoxy ou alcèneoxy et l'autre un reste alcoxy fluoré terminal de formule II.

8. Composés selon l'une des revendications 1 à 7, caractérisés en ce qu'ils se présentant sous forme de diastéréomères.

9. Procédé pour la fabrication de dérivés glycéro-3(2)-phospho-L-sériniques de formule générale I définis dans la revendication 1, caractérisé en ce qu'on prépare:

a) un dérivé acide glycéro-3(2)-phosphorique de formule générale

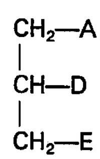

IV

dans laquelle A est défini comme dans la formule I, un des deux restes D et E a une des significations indiquées pour A dans la formule I ou bien est l'hydrogène et l'autre reste un groupement de formule générale

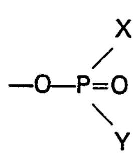

V

dans lequel X et Y, sont soit identiques et signifient un hydroxy ou un halogène, ou bien Y représente un groupement alcoxy ou aryloxy inférieur, sous réserve qu'au moins un reste A, D ou E signifie un groupement alcoxy ou un alcèneoxy de 5 à 30 atomes de carbone,

ou de son sel avec un dérivé L-sérinique protégé de formule générale

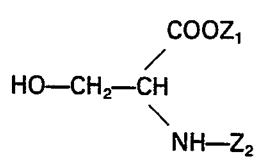

VI

dans laquelle $Z_1$ représente un groupe carboxyle et $Z_2$ un groupe amino de protection, transformé avantageusement en présence d'un moyen de condensation, on sépare finalement les groupes $Z_1$ et $Z_2$ de protection simultanément ou dans un ordre quelconque et on saponifie le cas échéant l'ester ou l'halogénure de l'acide glycérophosphorique obtenu, ou bien:

b) on prépare un ester de l'acide glycéro-3(2)-phosphorique de formule générale

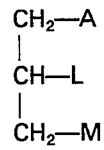

VII

dans laquelle A est défini comme dans la formule I, un des deux restes L et M a une des significations de A indiquées dans la formule I ou bien est l'hydrogène et l'autre reste un groupement de formule générale

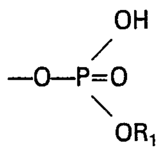

VIIIa

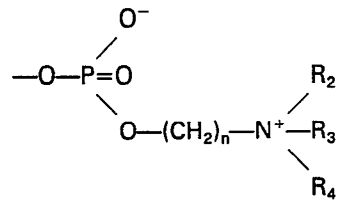

VIIIb

dans laquelle

$R_1$ est un alkyle de 1 à 6 atomes de carbone substitué le cas échéant par un groupe hydroxy ou halogène,

$R_2$, $R_3$ et $R_4$ sont identiques ou différents, indépendant les uns des autres, représentent l'hydrogène ou un méthyle et n étant un nombre entier compris entre 1 et 6, sous réserve qu'au moins un reste A, L ou M est un groupe alcoxy ou alcèneoxy de 5 à 30 atomes de carbone, avec la L-sérine en présence de phospholipase D pour provoquer la réaction et isolé les produits obtenus de formule générale I ou de leurs sels, et:

c) selon le cas, transformer en un sel pharmaceutiquement acceptable un composé de formule générale I obtenu selon l'une des variantes de préparation a) et b) ou d'un sel pharemaceutiquement non acceptable.

10. Préparation pharmaceutique comprenant des composés de formule générale I selon la revendication 1 ou un sel en combinaison avec des substances adjuvantes et/ou de traitement galéniques habituelles.

**Claims**

1. Glycero-3(2)-phospho-L-serine derivatives of the general formula

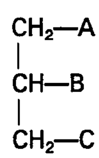

I

in which

A denotes ($C_5$—$C_{30}$)-alkoxy which is unsubstituted, or monosubstituted or polysubstituted by halogen, hydroxyl, alkoxy or cyano, or ($C_5$—$C_{30}$)-alkenoxy which is unsubstituted, or monosubstituted or polysubstituted by halogen, hydroxyl, alkoxy or cyano, where a double bond of the alkenoxy radical does not start at the C atom bound to the oxygen, or halogen or a group of the general formula

$$-O-(CH_2)_n-CF_3 \qquad \text{II}$$

in which

n stands for 0 or an integer 1, 2 or 3, and one of the two radicals

B and C, which is identical to A or different therefrom, has one of the meanings given for A or denotes hydrogen and the other radical in each case represents the phosphatidyl-L-serine group of the formula

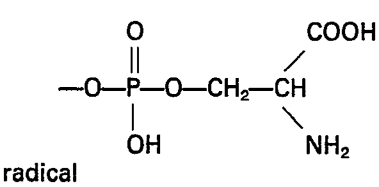

III

with the proviso that at least one radical

A, B or C denotes ($C_5$—$C_{30}$)-alkoxy or ($C_5$—$C_{30}$)-alkenoxy.

2. Compounds according to Claim 1, in which at least one radical A, B or C denotes ($C_{16}$—$C_{18}$)-alkoxy or ($C_{16}$—$C_{18}$)-alkenoxy.

3. Compounds according to Claim 1, in which one radical A, B or C denotes chlorine or fluorine.

4. Compounds according to one of Claims 1 to 3, in which C denotes the phosphatidyl-L-serine group of the formula III, one of the radicals A and B denotes alkoxy or alkenoxy and the other in each case denotes halogen.

5. Compounds according to one of Claims 1 to 3, in which B denotes the phosphatidyl-L-serine group of the formula III, one of the radicals A and C denotes alkoxy or alkenoxy and the other in each case denotes halogen.

6. Compounds according to one of Claims 1 or 2, in which A denotes alkoxy or alkenoxy, B denotes hydrogen or alkoxy and C denotes the phosphatidyl-L-serine group of the formula III.

7. Compounds according to one of Claims 1 or 2, in which C denotes the phosphatidyl-L-serine group of the formula III, one of the radicals A and B denotes alkoxy or alkenoxy and the other in each case denotes a terminally fluorinated alkoxy radical of the formula II.

8. Compounds according to one of Claims 1 to 7, characterized in that they exist as diastereomers.

9. Process for the preparation of glycero-3(2)-phospho-L-serine derivatives of the general formula I defined in Claim 1, characterized in that

a) a glycero-3(2)-phosphoric acid derivative of the general formula

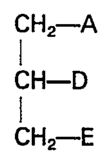 IV

in which

A is as defined in formula I, one of the two radicals

D and E has one of the meanings given in formula I for A or is hydrogen and the other radical in each case represents a group of the general formula

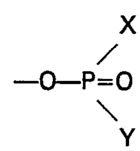 V

in which

X and Y are either identical and denote hydroxyl or halogen or Y stands for lower alkoxy or aryloxy, with the proviso that at least one radical A, D or E denotes ($C_5$—$C_{30}$)-alkoxy or ($C_5$—$C_{30}$)-alkenoxy, or its salts is reacted with a protected L-serine derivative of the general formula

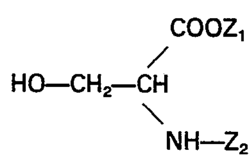 VI

in which

$Z_1$ denotes a carboxyl protective group and

$Z_2$ denotes an amino protective group, preferably in the presence of a condensing agent, then the protective groups $Z_1$ and $Z_2$ are removed in any sequence or simultaneously and, if appropriate, resultant glycerophosphoric acid esters or halides are hydrolyzed, or

b) a glycero-3(2)-phosphoric acid ester of the general formula

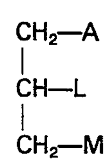 VII

in which

A is as defined in formula I, one of the two radicals

L and M has one of the meanings given in formula I for A or is hydrogen and the other radical in each case represents a group of the general formula

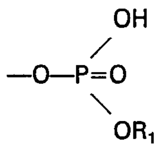

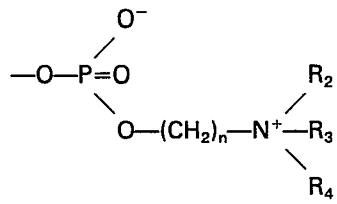

VIIIa or VIIIb

in which

$R_1$ denotes ($C_1$—$C_6$)-alkyl optionally substituted by hydroxyl or halogen,

$R_2$, $R_3$ and $R_4$, which are identical or different, independently of one another denote hydrogen or methyl and

n denotes an integer from 1—6 with the proviso that at least one radical A, L or M denotes ($C_5$—$C_{30}$)-alkoxy or ($C_5$—$C_{30}$)-alkenoxy, is made to react with L-serine in the presence of phospholipase D and resultant compounds of the general formula I or their salts are isolated, and

c) if desired, a compound of the general formula I obtained by process variant a) or b) or a pharmaceutically unacceptable salt thereof is converted into a pharmaceutically acceptable salt.

10. Pharmaceutical preparations containing compounds of the general formula I according to Claim 1 or a salt thereof in combination with customary pharmaceutical auxiliaries and/or excipients.